# EUROPÄISCHE PATENTSCHRIFT

(11) **EP 0 726 141 B1**
(45) Veröffentlichungstag und Bekanntmachung des Hinweises auf die Patenterteilung: **28.04.1999**
(21) Anmeldenummer: 96101173.1
(22) Anmeldetag: 29.01.1996
(51) Int. Cl.: B32B 27/32, C08K 3/34, A61F 13/02

(54) **Verwendung einer coextrudierten, matten Polyolefinfolie mit asymetrischem Schichtenaufbau und minimaler Rollneigung als Trägerfilm für Wundpflaster**
Use of a coextruded malt low-cockling polyolefin film with asymmetrical layer sequence as carrier film in adhesive plasters
Utilisation d'une feuille coextrudée mate en polyoléfine ayant une structure en couches asymmétrique et une faible tendance au gondolage comme feuille porteuse pour pansement

(30) Priorität: 10.02.1995 DE 19504318
(43) Veröffentlichungstag der Anmeldung: 14.08.1996
(73) Patentinhaber: WOLFF WALSRODE AG, 29655 Walsrode (DE)
(72) Erfinder: Guenter, Eger, D-29699 Bomlitz (DE); Schultze, Dirk, Dr., D-29683 Fallingbostel (DE)
(74) Vertreter: Braun, Rolf, Dr.

(56) Entgegenhaltungen:
- EP-A- 0 301 878
- US-A- 4 528 235
- US-A- 4 567 089

## Beschreibung

Die Erfindung bezieht sich auf die Verwendung einer rollneigungsarmen, mehrschichtigen, mindestens zweischichtige Polyolefinfolien mit asymetrischem Schichtenaufbau, die sich durch Coextrusion herstellen lassen und ohne weitere Behandlungsschritte im Herstellungsprozeß direkt als matte Folien anfallen. Die Folien bestehen aus Olefinpolymeren oder auch Olefin-Copolymeren und enthalten in einer der Aussenschichten größere Mengen an Schichtsilikat. Bedingt durch die unerwartete ähnliche Volumenkontraktion der gefüllten und ungefüllten Schichten der mindestens zweischichtigen Folie während des Herstellungsprozesses weisen die erfindungsgemäßen Folien eine äußerst geringe Rollneigung auf. Zusätzlich können sie Anteile an Verarbeitungshilfsmitteln, Farbpigmenten und Stabilisatoren enthalten. Durch ihre seidig matte Oberflächencharakteristik eignen sich diese Folien für die glanzarme Abdeckung von Oberflächen. Sie finden Verwendung als Trägerfolien von Wundpflastern, Heftpflastern oder Pflaster-Strips.

Die erfindungsgemäßen verwendeten Folien eignen sich für eine Vielzahl von Anwendungen, wobei hier auf die Verwendung als Trägerfolien für Wundpflaster abgehoben wird. Wundpflaster bestehen im allgemeinen aus einem Trägermaterial, beschichtet mit einer Haftklebemasse und einer Wundabdeckung, die flächenmäßig kleiner als das Trägermaterial ist sowie einem Releasematerial, welches während der Lagerung sowohl Haftklebstoff als auch Wundabdeckung schützt. Folien für derartige Anwendungen müssen eine Vielzahl von Anforderungen erfüllen, wobei neben den technischen vor allem ästhetische Wünsche zu befriedigen sind.

Folien für medizinische Pflaster müssen formstabil sein, sich chemisch oder physikalisch vorbehandeln lassen um bedruckt und/oder beschichtet zu werden. Sie sollen zudem mechanische Eigenschaften besitzen, die eine geringe Rollneigung, eine minimale Steifigkeit, beispielsweise charakterisiert durch geringe Dehnkräfte bei 10% Dehnung und minimale Rei ßkräfte >15 N/15mm Prüfstreifenbreite umfassen. Die geringe Rollneigung stellt ein verarbeitungs- und anwendungstechnisches Erfordernis dar. Bei ausgeprägter Rollneigung können klebstoffbeschichtete Teile der Pflaster im Zuge gängiger Transferkaschierungsverfahren gegeneinander verkleben.

Zu den ästhetischen Anforderungen gehört in erster Linie eine seidig-matte Oberfläche, die ein hautähnliches Erscheinungsbild gewährleistet. Matte Oberflächen werden allgemein durch den Glanz beurteilt, minimale Glanzwerte werden angestrebt. Für Pflaster haben sich allgemein Glanzwerte < 5 bewährt, gemessen nach DIN 67 530 unter einem Winkel von 20°.

Auch die Haptikder Pflasterfolien muß ansprechend sein, hierzu zählt insbesondere ein weicher Griff.

In der Anwendung eines Pflaster-Zuschnitts bleibt die Belastung durch Zug-Kräfte praktisch gering, da der Haftkleber eine besonders geringe Klebehaftung zu einem üblicherweise eingesetzten Trennmaterial wie beispielsweise silikonisiertes Papier oder steife Folien aufweist. Pflaster-Zuschnitte unterscheiden sich hier von Rollenpflastern, bei denen höhere Abzugskräfte auftreten können, da hier auf eine Trennschicht verzichtet wird und die Klebschicht auf die Außenseite des Pflasters zuliegen kommt.

Ebenso handelt es sich bei Folien für Rollenpflaster nicht um sehr weiche Materialien. Bei der Längszug-Belastung, wie sie z.B. bei der Entnahme als Klebeband von der Rolle auftritt, ist eine geringe Längs-Dehnung erwünscht und allgemein eingestellt.

Insofern sind zur Charakterisierung eines geeigneten Folienmaterials zur Verwendung als Trägerfilm für Pflaster-Zuschnitte nicht nur die hohen Reißkraft- bzw. Reißdehnungswerte maßgeblich, sondern stellen außerdem die Anfangsdehnungwerte bei Zugbelastung wichtige Beurteilungskriterien dar.

In der Vergangenheit wurden vielfach matt-kalandrierte Polyvinylchlorid (PVC)-Folien als Trägerfilme eingesetzt. Die öffentliche Diskussion über die gesundheitlichen Auswirkungen des Monomeren Vinylchlorid und der sogenannten äußeren Weichmacher, wobei insbesondere die Phthalat-Weichmacher ins Zentrum der Kritik rückten, verstärkten den Druck zur Substitution von Weich-PVC.

Die Anforderungen an Folien für medizinische Pflaster sind beispielsweise in den deutschen Gebrauchsmusteranmeldungen G 90 12 161.9 und G 93 06 768.2 (beide Beiersdorf) dargestellt. Die Klassifizierung von Pflastern ist durch die jeweiligen Arzneibücher geregelt, in Deutschland ist dies beispielsweise das DAB 10 (1991).

Die Mattierungsverfahren, die zum Stand der Technik gehören, umfassen vor allem Präge- und Beschichtungstechniken, bei denen die Folie i.a. an gravierten oder anderweitig mattierten Walzen vorbeigeführt wird und unter Druck die gewünschte Oberflächenstruktur eingeprägt wird. Diese Technik unterliegt jedoch gewissen Beschränkungen. Insbesondere von wirtschaftlichem Interesse ist, daß Prägewerkzeuge sich nur mit geringen Geschwindigkeiten betreiben lassen, die deutlich geringer als die der Folienherstellungsanlagen liegen. Zudem sind der Prägung durch die Relaxation der auf die zu prägenden Materialien gepressten Strukturen und die Gravurtiefen Grenzen gesetzt. Mattbeschichtete Folien neigen zu einer deutlichen Rollneigung, die vor allem quer zur Bahnrichtung nicht durch eine Anpassung von Verfahrensparametern wie Vor- oder Bahnspannung begrenzt werden kann. Bei solchen Verfahren werden i.a. Folien mit deutlich anisotropen Eigenschaften in der Folienebene erhalten.

Ein weiterer Nachteil des Prägens ist, daß ein auf einem thermoplastischen Kunststoff aufgebrachtes Prägebild unter Wärmeeinfluß relaxieren kann, da aufgrund der Fließfähigkeit der Harze diese dem Bestreben nachgeben die Grenzfläche zu minimieren. Eine derartige Glättung der Oberfläche bewirkt eine deutlichere Reflexion von Licht und vermindert somit den Mattigkeitseindruck. Thermische Belastungen der Folien können bei nachfolgenden Bearbeitungsschritten vielfach auftreten. Typische Konfektionierungsschritte, bei denen thermische Belastungen auftreten können, sind beispielsweise das Stanzen mit beheizter Klinge oder das Perforieren.

Ein einseitig mattierter Aufbau hat sich bei geprägten Folien bewährt, die im Zuge einer Transferkaschierung mit einem Haftklebstoff beschichtet wurden. Auf der glatten Seite einer mattierten Polyolefinfolie ließen sich gute Haftungswerte für die Klebstoffverankerung erreichen.

Ein über ein Mattierungsmittel in-line erzeugter Matteffekt der Trägerfolie, der den komplexen Anforderungen an Pflaster genügt und zudem fertigungstechnische Vorteile besitzt, ist noch nicht angetroffen worden. Aus der Patentliteratur sind vereinzelt Verfahren zur in-line-Rezeptur-Mattierung bekannt. Die dort beschriebenen Rezepturen genügen aber nicht den Anforderungen an Trägerfilme für Pflaster.

So beschreibt die japanische Patentschrift 3216-722 (Mitsubishi Petrochemical) Mischungen aus EVA-Copolymeren und Ethylen-alpha-Olefin-Copolymeren zur in-line-Mattierung. Mit solchen Mischungen lassen sich aber nicht die für medizinische Pflaster geforderten geringen Glanzwerte erzielen. In asymetrischen Folienaufbauten zeigen solche Rezepturen jedoch eine zu deutliche Rollneigung. Dies läßt sich auch aus Tabelle 1 ersehen, in der die Rollneigung für die Folien aus den Vergleichsbeispielen angegeben ist.

Auch mit anorganischen Materialien hochgefüllte Filme sind bereits bekannt, so beschreibt die PCT/US93/07922 (GAIA Research L.P.) die durch einen hohen anorganischen Füllgrad verbesserte biologische Abbaubarkeit einer einschichtigen Folie. Eine solche Folie besitzt jedoch zwei identische Oberflächen, wenn sie keiner weiteren Behandlung unterzogen wird. Zudem beeinträchtigen die hohen Füllgrade jenseits des Perkolationspunktes die mechanische Festigkeit einer solchen Folie. Die für die gute biologische Abbaubarkeit ursächliche hohe Anzahl an potentiellen Fehl- und Angriffsstellen ist zudem für medizinische Pflaster indiskutabel. Bei erforderlicher steriler Wundabdeckung ist eine Fehlstellen-freie Folie unabdingbar.

Die EP 0 347 745 (Procter & Gamble) beschreibt derart hoch gefüllte Foliensysteme, daß sich diese durch mindestens eine weiße Seite auszeichnen. Solch hohe Füllgrade an farbstarken Pigmenten sind für Pflasterfolien unerwünscht, da sie im Falle tranzluzenter Ausführung ein Durchscheinen der darunterliegenden Hautpartien verhindern. Bei farbiger Ausführung wird der Farbeindruck stark ins milchig-gräuliche verschoben, so daß keine Ware Farbgebung zu erzielen ist. Die dort beschriebenen Schichtdickenkombinationen lassen zudem nicht die Herstellung dünner mattierter Folien zu, da die Größe der zur Erzielung des Matt-Effektes notwendigen Teilchen-Korngrößen die maximale beschriebene Schichtdicke übersteigt. Als geeignete Füllstoffe werden dort Ti0₂, CaC0₃, Si0₂ und farbige Pigmente beschrieben.

Die PCT/US92/07736 (Nashua) beschreibt mehrschichtige Folien für Klebeband-Anwendungen, bei denen eine Schicht aus einem wesentlich steiferen Material als die zweite Schicht besteht. Zudem werden dort nur asymmetrische Pigmentbeladungen angestrebt. Folien des dort beschriebenen Aufbaues sind zu steif für Pflaster, da die dort beschriebenen Schichten aus HDPE nicht in der Lage sind, sich flexibel an die Bewegungen der überdeckten Haut im Wundbereich anzupassen.

Es galt eine einseitig mattierte Folie bereitzustellen, die ohne weitere mechanische Behandlungsschritte aus dem Folienherstellungsprozeß erhalten wird. Um die einfache Weiterverarbeitung zu Trägerfilmen für Pflaster-Zuschnitte zu gewährleisten, sollte die benötigte Folie rollneigungsarm sein. Aus ökologischer Sicht sollte die Folie frei von Halogenverbindungen und aromatischen Weichmachern mit vergleichsweise geringem Molekulargewicht sein. Sie sollte ein glatte Seite aufweisen, so daß sie sich auf den gängigen Transferkaschierungsmaschinen problemlos mit einem Haftklebstoff beschichten läßt. Zudem sollte die Folie so elastisch einstellbar sein, daß sie sich flexibel an Hautbewegungen im Wundbereich anpassen kann. Weitestgehend isotrope Eigenschaften in der Folienebene sind für einen möglichst universellen Einsatz der Folie von Vorteil.

Gelöst wurde diese Aufgabe durch eine zur Folie ausgeformte rollneigungsarme Kunststoff-Schichtstruktur, zu deren Eigenschaften eine angenehme Haptik, d.h. ein weicher Griff und eine einseitige Mattigkeit zählen. Die Folie ist frei von Halogenkohlenwasserstoffen und frei von äußeren Phthalat-Weichmachern. Sie besteht aus Olefin-basierenden Kunststoffen, deren Weichheit sich gegebenenfalls durch Copolymerisation mit weiteren Olefinen oder Sauerstoff- Atome enthaltende ungesättigte Monomere einstellen läßt, beispielweise seien Vinylverbindungen wie Vinylacetat und Acrylverbindungen wie Butylacrylat genannt. Bevorzugt lassen sich ethylenische Copolymerisate des Vinylacetats, Butylacrylats oder Methylacrylats zur Herstellung der Folie einsetzen.

[0021 ] Die erfindungsgemäß eingesetzte Folie wird bevorzugt durch Verarbeitung aus der Schmelze erhalten, wobei die unterschiedlichen Schichten nur geringfügige Unterschiede in ihrer Volumenkontraktion beim Abkühlen aufweisen, so daß ein rollneigungsarmer Aufbau erhalten wird.

Die Schichtstruktur wird aus mindestens einer Schicht (1) aus einem Polyolefin und/oder einem olefinischen Copolymer und/oder Ethylen-Terpolymer und/oder deren Mischungen, gegebenenfalls geschieht dies unter Zusatz geeigneter Farben und Stabilisations-Additive in wirkungsvollen Mengen und mindestens einer zweiten mattierten Schicht (2) gebildet. Die Schicht (2) ist dadurch charakterisiert, daß diese als Matrix, d.h. mit überwiegendem Anteil, Polymere mit den gleichen wesentlichen Merkmalen wie Schicht (1) aufweist und die Mattierung durch Zuschlag eines Schichtsilikats erhält. Als Matrixmaterial für die Schicht (2) wird bevorzugt das jeweils gleiche Polymer wie für Schicht (1) verwendet.

Die für die Folien bevorzugten Olefin-Co- oder Olefin-Homo-Polymerisate besitzen Dichten zwischen 0,900 und 0,960 g/cm³. In besonderem Maß eignen sich Olefin Polymerisate mit Dichten zwischen 0,915 und 0,930 g/cm³. Besonders bevorzugt kommen Olefin-Polymerisate mit Dichten zwischen 0,920 und 0,930 g/cm³ zum Einsatz.

Die als Matrixmaterialien geeigneten Polymerisate besitzen bevorzugt Viskositäten, die durch Schmelzflußindices zwischen 0,2 und 8,0 g/10 min, gemessen nach DIN 53 735 bei 190°C und einer Prüfbelastung von 2,16 kg, gekennzeichnet sind. Besonders bevorzugt werden Matrixmaterialien mit Schmelzflußindices, die unter den o.g. Bedingungen zwischen 1,0 und 4,0 g/10 min liegen.

Olefin-Copolymerisate mit Sauerstoff-Atome enthaltenden Comonomeren weisen erfahrungsgemäß eine schnellere Verankerungszeit des Haftklebstoffs bei der Transferkaschierung auf.

Der Schichtsilikat-Anteil der mattierten Schicht der erfindungsgemäß eingesetzten Folie sollte vorteilhafterweise mindestens 10 Gew.-% betragen, damit eine deutliche Mattierung erzielt wird und andererseits 25 Gew.-% nicht übersteigen. So erfolgt erstaunlicherweise keine merkliche Beeinträchtigung der mechanischen Festigkeiten.

Überraschenderweise besitzen solche asymetrischen Strukturen, die einseitig durch einen hohen Anteil an Schichtsilikat ausgezeichnet sind, nur minimale Unterschiede in ihrem Abkühlverhalten nach der Verarbeitung aus der Schmelze zu der nicht ausgerüsteten Seite. Diese erstaunlich geringen Unterschiede im thermischen Ausdehnungsverhalten der hochgefüllten und der nichtgefüllten Seite der asymmetrischen Struktur führen zu geringsten Spannungen zwischen den beiden Seiten und damit zu einer höchstens minimalen Rollneigung einer Folie mit einem derartigen Aufbau.

Die zur Mattierung eingesetzten Schicht- oder auch Phyllosilikate sind auf atomarer Ebene durch ein Silicium: Sauerstoff-Verhältnis von 4 : 10 in den Silikat-Schichten gekennzeichnet. Ihre Si0₄-Tetraeder sind derart aufgebaut, daß drei der vier Sauerstoffatome auch Nachbartetraedern angehören, so daß ein zweidimensionales Netzwerk entsteht. Die Blattstruktur der Schichtsilikate ist die Ursache für ihr gutes Gleitvermögen und ihre Hydrophobie. Diese Charakteristika sind wiederum die Ursache für die ausgewogenen Füllstoff-Eigenschaften der Schichtsilikate in Polyolefinen.

Die Schichtsilikate kommen natürlich vor, zur Kompensation der Anionen-Ladungen der S'₄0₁₀ ⁴--Einheiten trifft man vor allem die Kationen des Kalium, Magnesium und Aluminium an. Kennzeichnend für das natürliche Vorkommen ist auch die häufig anzutreffende Substitution der vorgenannten Kationen durch Kationen anderer Metalle. Hier sind insbesondere die Kationen des Eisen, Mangan und Calcium, aber auch des Lithium und Natrium anzutreffen.

Für die Mattierung geeignete Schichtsilikat-Partikel besitzen eine ausreichende Größe, um der Oberfläche der hier beschriebenen Folien eine Rauhtiefe von 10 µm zu verleihen. Hierzu sind Teilchengrößen oberhalb 10 µm günstig. Bei zu kleinen Korngrößen um ca. 2 µm, die i.a. als hochfein bezeichnet werden, wird in erster Linie die Kristallitbildung angeregt und keine ausreichende Mattierung erzielt. Bei zu großen Partikeln erhält die Folie eine grobe, unangenehme Haptik. Als ebenfalls günstig haben sich deshalb maximale Teilchengrößen von 20 I-Lm erwiesen. Bei Berücksichtigung der üblichen Größenverteilungen wird eine 98 % Fraktion unterhalb 20 µm Teilchendurchmesser als geeignet im Sinne der o.g. maximalen Teilchengröße angesehen. Zur Klassifizierung der Teilchengröße wird im allgemeinen die durch Siebanalyse ermittelte Korngröße gewählt. Die

Definition des Begriffes Korngröße und eine Beschreibung einer Siebanalyse erfolgen u.a. in der DIN 53 477.

Bevorzugt ist der Einsatz von Talkum oder auch kurz als Talk bezeichnet, einem Schichtsilikat der idealen Zusammensetzung Mg3(OH)2Si4010. Talkum kommt natürlich in vier Teilchenformen vor: faserig, plättchenartig, nadelförmig und modular bzw. steatitisch. Für den erfindungsgemäßen Einsatz besitzt die plättchenartige Form Vorteile. Beim Talkum befinden sich Oxid und Hydroxid des Magnesium gesandwicht zwischen zwei Siliciumoxid-Struktureinheiten mit Blattstruktur. Die Zusammensetzungen von Talkum-Qualitäten sind beispielhaft u.a. von Evans in: Einführung in die Kristallchemie, Walter de Gruyter, Berlin 1976, oder von Bosshard und Schlumpf im: Taschenbuch der Kunststoff-Additive, Gächter und Müller (Hrsg.), Carl Hanser Verlag, München 1983, beschrieben worden.

Talkum besitzt keine deutliche farbgebende Wirkung, für den Fachmann nicht naheliegend war, daß der transluzente Charakter der Polyolefine erhalten wird und sich nur geringfügige Trübung beobachten läßt. Entsprechende Vergleichswerte für die Talkum-mattierte Folien sind in der vergleichenden Übersicht der Eigenschaften der im Rahmen der Beispiele und Vergleichsbeispiele hergestellten Folien in Tabelle 1 wiedergegeben.

Durch die Variationsmöglichkeiten bei der Wahl der olefinischen Basisharze lassen sich die mechanischen Eigenschaften der Folie wie Flexibilität und Elastizität weitestgehend variieren. Für großflächige Wundabdeckungen können bevorzugt steifere Einstellungen gewählt werden. Während für Pflaster, die im Bereich der Gelenke eingesetzt werden sollen, elastischer eingestellte Folien angenehmere Trageeigenschaften besitzen.

Es ist im allgemeinen von vor allem wirtschaftlichen Vorteil, wenn die mattierte Schicht einen geringen Anteil an der Gesamtschichtdicke der einseitig mattierten Folie aufweist. Von besonderem Interesse sind deshalb Dicken-Kombinationen, in denen der Anteil der mit Schichtsilikat mattierten Schicht 15-40% der Gesamtschichtdicke beträgt.

Die Zugabe des mattierenden Schichtsilikats erfolgt bevorzugt in Form eines Additiv-Masterbatches, welches in einer besonders bevorzugten Ausführungsform einen Gewichtsanteil an Schichtsilikat zwischen 30 und 80 Gew.-% aufweist. Ein geringerer Anteil besitzt wirtschaftliche Nachteile, während ein höherer Anteil an Schichtsilikat im Masterbatch eine schlechte Verteilung mit sich bringt, die sich beispielsweise in unerwünschten Silikat-Agglomeraten zeigt. Dies bedingt eine ungleichmäßigere Rauhigkeit und damit unangenhmere Haptik.

Für die Herstellung solcher Additiv-Masterbatche haben sich Olefin-Polymere oder Olefin-Copolymere mit vergleichsweise hohen Schmelzflußindices zwischen 8 und 24 g/10 min, gemessen nach DIN 53 735 bei 190°C und einer Prüfbelastung von 2,16 kg, bewährt. Um die Masterbatch-Homogenität zu verbessern können gegebenenfalls noch weitere Plastifizierungshilfsmittel wie beispielsweise Wachse enthalten sein. Zur Verhinderung einer thermischen Schädigung der Olefin-Komponente des Masterbatches werden letztere üblicherweise mit Stabilisatoren ausgerüstet.

In einer bevorzugten Ausführung können einzelne oder alle Schichten der mehrschichtigen Folie durch weitere Zugabe von Verarbeitungshilfen, Füllstoffen, Farben und Stabilisatoren modifiziert sein. Farben ermöglichen die Herstellung von Pflastern für spezielle Einsatzgebiete.

In einer dieser ausgewählten Ausführungformen enthält mindestens eine der Schichten Farbpigmente. Beige eingefärbte Folie ist beliebt, wenn Wunde und Wundabdeckung nicht offen sichtbar sein sollen. Bunte Einfärbungen und Druckapplikationen finden vielfach Eingang in Kinderpflaster. Weitere Additive wie Silikate und Wachse modifizieren die anwendungstechnischen Eigenschaften, insbesondere das Gleitverhalten. Stabilisatoren ermöglichen es, die erfindungsgemäßen Folien über einen längeren Zeitraum zu erhalten bzw. Schädigungen bei der Verarbeitung zu vermeiden. Die gängigen Additive für Kunststoffe sind von Gächter und Müller im: Handbuch der Kunststoff Additive, Hanser Verlag, München 1983, beschrieben.

Die erfindungsgemäß eingesetzten Folien lassen sich nach den gängigen Folien-Co-Extrusions-Verfahren herstellen. Dies sind Blas- und Flachfilmprozesse. Diese Prozesse sind beispielsweise von Michaeli in: Extrusionswerkzeuge für Kunststoffe und Kautschuk, Carl Hanser Verlag, München 1991, beschrieben. Weiterführende Informationen gibt das Handbuch der Kunststoff-Extrusionstechnik, herausgegeben von Hensen, Knappe und Potente, Carl Hanser Verlag, München 1989. Als besonders geeignet für deren Herstellung hat sich der Blasfolienprozeß erwiesen. Hier wird die Schmelze von einer Ringdüse abgezogen, der erhaltene Schlauch i.a. durch einen gegenüber der Umgebung erhöhten Innendruck aufgeweitet, schließlich flachgelegt, abgequetscht, aufgetrennt und gewickelt.

Eine besondere Ausführungsform der erfindungsgemäß eingesetzten Folien zeichnet sich dadurch aus, daß die Folie eine Gesamtschichtdicke zwischen mindestens 40 I-Lm und höchstens 200 am aufweist. Besonders geeignet sind Folien mit mindestens 50 µm und höchstens 100 I-Lm Dicke.

Die erfindungsgemäß eingesetzten Folien können im Hinblick auf ihre spätere Verwendung durch ein Behandlungsverfahren in ihren Oberflächeneigenschaften modifiziert werden. Hierzu eignen sich insbesondere übliche Corona-, Plasma- oder Fluoraber auch Flammbehandlungen. Solche Verfahren wurden beispielsweise von Dorn und Wahono in: Maschinenmarkt 96 (1990) 34-39 oder Milker und Möller in: Kunststoffe 82 (1992) 978-981 ausführlich dargestellt. Ein bevorzugtes Verfahren ist die Corona-Behandlung.

Bei der Corona-Behandlung wird zweckmäßigerweise so vorgegangen, daß die Folie zwischen zwei als Elektroden dienenden Leiterelementen hindurchgeführt wird, wobei zwischen den Elektroden eine so hohe Spannung - üblicherweise Wechselspannung von etwa 10 kV mit einer Frequenz von 10 kHz - anliegt, daß Sprüh- oder Coronaentladungen stattfinden können. Durch diese Entladungen wird die Luft entlang der Folienoberfläche ionisiert, so daß es zu Reaktionen an der Folienoberfläche kommt, bei denen im Vergleich zu der Polymermatrix polarere Gruppen entstehen. Die für die Vorbehandlung der Folien benötigten Behandlungsintensitäten liegen hierbei im üblichen Rahmen, wobei Behandlungsintensitäten bevorzugt sind, die Oberflächenspannungen von 38 - 50 mN/m ergeben.

Die erfindungsgemäß verwendeten Folien können ein- oder beidseitig vorbehandelt werden. Solche Behandlungen dienen der Verbesserung der Oberflächen hinsichtlich ihrer Haftungseigenschaften zu Druck- und/oder Beschichtungsmaterialien. Bei Pflastern übliche Druckapplikatonen sind beispielsweise bunte Tiere oder Gegenstände, die bevorzugt auf die mattierte Seite von Kinderpflastern gedruckt werden. Die Beschichtung mit Haftklebstoffen ist ein bei Pflastern allgemeinübliches Verfahren, sie erfolgt bevorzugt auf der glatten Folienseite.

Im folgenden wird die Erfindung anhand von Beispielen und Vergleichsbeispielen noch näher erläutert, ein Vergleich der Beispiele untereinander und mit den Vergleichsbeispielen erfolgt anhand von der in Tabelle 1 dargestellten Übersicht relevanter Eigenschaften.

### Beispiel 1

Im Rahmen des ersten Beispiels wurde durch Coextrusion auf einer Blasfolienanlage eine zweischichtige Folie hergestellt. Die zum Aufschmelzen eingesetzten Extruder wurden mit Temperaturprogrammen von 160-190°C betrieben, die Temperatur des Blasfolienwerkzeugs lag bei 190°C.

Die ausgeformte Folie hatte eine Schichtdickenabfolge 20 µm, 60 µm. Aufgrund der durch die aufrauhende Wirkung der erfindungsgemäßen Zugabe des Schichtsilikats hervorgerufenen Ungenauigkeit einer durch mechanische Abtastung ermittelten Dicke, sind die angegebenen Dicken Nennschichtdicken, die rechnerisch unter der Annahme einer glatten Oberfläche der real rauhen, mattierten Seite ermittelt wurden. Auf Basis der Dichten der Rohstoffe bzw. der gemittelten Dichten der Rohstoff-Mischungen lassen sich diese Nennschichtdicken bzw. die Gesamtnennschichtdicke durch Addition der für die jeweiligen Schichten erhaltenen Werte errechnen .

Die nicht-mattierte 60 µm dicke Schicht, die bei der Folienherstellung die Aussenschicht der Schlauchblase bildete, wurde aus einem Ethylen-Vinyl-Acetat (EVA)- Copolymer mit einem VA-Anteil von 5 Gew.-% gebildet. Das EVA-Harz hatte einen Schmelzflußindex (MFI) von 7,5 g/10 min, gemessen nach DIN 53 735 bei 190 °C mit einer Prüfbelastung von 2,16 kg, und eine Dichte von 0,928 g/cm³. Das EVA-Polymerisat wurde mit einer Massetemperatur von 193 °C verarbeitet. Die von dem reinen EVA-Harz gebildete Aussenschicht wurde nach der Folienherstellung einer Corona-Behandlung unterzogen und eine Oberflächenspannung von 40 mN/m erzielt.

Die 20 µm dicke Innenschicht der Folie wurde aus einer Mischung bestehend aus 70 Gew.-% des für die 60 µm dicke Schicht eingesetzten EVA-Copolymeren und 30 Gew.-% eines Schichtsilikat-Masterbatches hergestellt. Dies Masterbatch hatte einen Talkum-Anteil von 50 Gew.-% in einer Low-Density-Polyethylen-Matrix. Die Dichte des Masterbatches betrug 1,39 g/cm³. Die Größe der Talkum-Teilchen war kleiner 22 am. Die Mischung aus EVA-Harz und Talkum-Masterbatch in der Innenschicht wurde mit einer Massetemperatur von 202 °C verarbeitet.

### Beispiel 2

Mit einem Coextrusions-Blasfolienwerkzeug analog zu dem für Beispiel 1 benutzten wurde eine zweischichtige Folie mit einer Nennschichtdickenabfolge 15 µm, 45 µm geformt. Die 45 µm dicke Schicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für diese Schicht verwendeten EVA-Copolymerisats, einem MFI von 1,8 g/1 0 min, gemessen nach DIN 53 735 bei 190 °C mit einer Prüfbelastung von 2,16 kg, und einer Dichte von 0,926 g/cm³ gebildet. Diese Schicht wurde auf dem Coextrusionsblaskopf als Aussenschicht gewählt. Das EVA-Polymerisat wurde mit einer Massetemperatur von 189 °C verarbeitet.

Die 15 µm dicke Innenschicht der Folie wurde aus einer Mischung bestehend aus 75 Gew.-% des für die 45 µm dicke Schicht eingesetzten EVA-Copolymeren und 25 Gew.-% eines Schichtsilikat-Masterbatches hergestellt. Das Gleitmittel-freie Masterbatch hatte einen Talkum-Anteil von 60 Gew.-% in einer Polyethylen-Matrix. Die Dichte des Masterbatches betrug 1,49 g/cm³. Die Größe der Talkum-Teilchen war kleiner 15 µm. Die Mischung in der Innenschicht wurde mit einer Massetemperatur von 194 °C verarbeitet.

Die Aussenseite der im Rahmen von Beispiel 2 hergestellten Folie wurde einer Corona-Behandlung unterzogen und so eine Oberflächenspannung der nicht mattierten Seite der Folie von 44 mN/m eingestellt.

### Beispiel 3

Unter Verwendung der in Beispiel 1 beschriebenen Verarbeitungsmaschine ließ sich eine coextrudierte Folie mit einer Schichtdickenabfolge 15 µm, 45 µm herstellen. Die 45 wm dicke Aussenschicht des Films wurde aus einem EVA-Basismaterial unter Zugabe eines Farbmasterbatches aufgebaut. Das EVA-Basismaterial hatte einen VA-Anteil von 5 Gew.-%, einen Siliciumdioxid-Anteil von 1000 ppm, jeweils bezogen auf die Gesamtmasse des für diese Schicht verwendeten EVA-Copolymerisats, einen MFI von 1,8 g/10 min, gemessen nach DIN 53 735 bei 190 °C mit einer Prüfbelastung von 2,16 kg, und eine Dichte von 0,926 g/cm³. Das hautfarbene Farbbatch wurde zu 9 Gew.-% dosiert. Es hatte eine Dichte von 1,81 g/cm³ und einen Schmelzflußindex von 9,2 g/10 min, gemessen nach DIN 53 735 bei 190 °C mit einer Prüfbelastung von 2,16 kg. Die Materialmischung wurde mit einer Massetemperatur von 188°C verarbeitet. Die aus diesen Materialien gebildete Aussenschicht wies nach Herstellung und Corona-Behandlung eine Oberflächenspannung von 40mN/m auf.

Die 15 µm dicke Innenschicht der Folie wurde aus einer Mischung bestehend aus 56 Gew.-% des für die 45 µm dicke Schicht eingesetzten EVA-Copolymeren, 9 Gew.-% des für die dickere Schicht eingesetzten Farbbatches und 35 Gew.-% eines Schichtsilikat-Masterbatches hergestellt. Das Masterbatch hatte einen Talkum-Anteil von 40 Gew.-% in einer Low-Density-Polyethylen-Matrix. Zusätzlich enthielt das Masterbatch 1,5 Gew.-% eines Amidwachses und 0,5 Gew.-% eines Stabilisators. Die Dichte des Masterbatches betrug 1,29 g/cm^{3.} Die Größe der Talkum-Teilchen war kleiner 20 pm. Die Mischung in der Innenschicht wurde mit einer Massetemperatur von 194 °C verarbeitet.

### Beispiel 4

Mit einem Coextrusions-Blasfolienwerkzeug analog zu dem für Beispiel 1 benutzten wurde eine eingefärbte zweischichtige Folie mit einer Nennschichtdickenabfolge 15 wm, 55 µm geformt. Die 55 µm dicke Aussenschicht der Folie wurde im wesentlichen aus einem EVA-Copolymer mit einem VA-Anteil von 5 Gew.-%, einem Erucasäureamid-Anteil von 300 ppm, einem Siliciumdioxid-Anteil von 1800 ppm, jeweils bezogen auf die Gesamtmasse des für diese Schicht verwendeten EVA-Copolymerisats, einem MFI von 2,0 g/10 min, gemessen nach DIN 53 735 bei 190 °C mit einer Prüfbelastung von 2,16 kg, und einer Dichte von 0,928 g/cm³ gebildet. Diesem Copolymerisat wurden 9 Gew.-% eines handelsüblichen beige-eingefärbten Masterbatches beigegeben. Das Farbbatch hatte eine Dichte von 1,64 g/cm3 und einen Schmelzflußindexvon 11,2 g/10 min, gemessen nach DIN 53 735 bei 190 °C mit einer Prüfbelastung von 2,16 kg. Die EVA-Polymerisat/Farbbatch-Mischung wurde mit einer Massetemperatur von 188 °C verarbeitet.

Die 15 µm dicke Innenschicht der Folie wurde aus einer Mischung bestehend aus 61 Gew.-% des für die 55 µm dicke Schicht eingesetzten EVA-Copolymeren, 9 Gew.-% des für die dickere Schicht eingesetzten fleischfarbenen Farbmasterbatches und 30 Gew.-% eines Schichtsilikat-Masterbatches hergestellt. Das Masterbatch hatte einen Talkum-Anteil von 50 Gew.-% in einer Low-Density-Polyethylen-Matrix. Die Dichte des Masterbatches betrug 1,39 g/cm^{3.} Die Größe der Talkum-Teilchen war kleiner 22 µm. Die Mischung in der Innenschicht wurde mit einer Massetemperatur von 195 °C verarbeitet.

Die Aussenseite der im Rahmen von Beispiel 4 hergestellten Folie wurde einer Corona-Behandlung unterzogen und so eine Oberflächenspannung der nicht mattierten Seite der Folie von 38 mN/m eingestellt.

### Beispiel 5

Mit einem Zweischicht-Coextrusionswerkzeug wurde eine Folie der Nennschichtdickenabfolge 40 µm, 40 µm hergestellt. In der Aussenschicht wurde eine Mischung aus 73 Gew.-% eines Ethylen-Methylacrylat (EMA) - Copolymeren, 7 Gew.-% eines hautfarbenen Farbmasterbatches und 20 Gew.-% eines Talkum-Masterbatches verarbeitet. Die Massetemperatur lag bei 194°C. Das EMA-Copolymer hatte einen MA-Anteil von 13 Gew.-%, einen MFI von 2,5 g/10 min, gemessen nach DIN 53 735 bei 190°C mit einer Prüfbelastung von 2,16 kg, eine Dichte von 0,96 g/cm³ und einen Gleitmittelanteil von 700 ppm Erucasäureamid. Das Farbbatch hatte eine Dichte von 1,64 g/cm3 und einen Schmelzflußindex von 11,2 g/10 min, gemessen nach DIN 53 735 bei 190 °C mit einer Prüfbelastung von 2,16 kg. Das Talkum-Additivbatch bestand zu 70 Gew.-% aus Schichtsilikat und zu 30 Gew.-% aus LDPE. Die mittlere Korngröße des eingesetzten Talkum war keiner 10 µm.

Die Innenschicht wurde aus 93 Gew.-% des auch für die Aussenschicht verwendeten EMA-Copolymers und 7 Gew.-% des ebenfalls für die Aussenschicht eingesetzten Farbbatches gebildet. Die Verarbeitungstemperatur der Schmelze dieser Mischung lag bei 193°C.

### Vergleichsbeispiel A

Mit einem Coextrusions-Blasfolienwerkzeug entsprechend Beispiel 1 wurde eine zweischichtige Folie mit einer Nennschichtdickenabfolge 20 µm, 60 µm geformt. Die 60 µm dicke Schicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für diese Schicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet. Diese Schicht wurde auf dem Coextrusionsblaskopf als Aussenschicht gewählt. Das EVA-Polymerisat wurde mit einer Massetemperatur von 191 °C verarbeitet.

[0061 ] Die 20 µm dicke Innenschicht der Folie wurde aus einem Blend bestehend aus 80 Gew.-% des für die 60 µm dicke Schicht eingesetzten EVA-Copolymeren und 20 Gew.-% eines Propylenhomopolymerisats mit einer Dichte von 0,905 g/cm³ und einem MFI von 3,3 g/10 min, gemessen nach DIN 53 735 bei 230°C mit einer Prüfbelastung von 2,16 kg, hergestellt. Das Polymer-Blend in der Innenschicht wurde mit einer Massetemperatur von 204 °C verarbeitet.

Die Aussenseite der im Rahmen von Vergleichsbeispiel A hergestellten Folie wurde einer Corona-Behandlung unterzogen und so eine Oberflächenspannung der nicht mattierten Seite der Folie von 42 mN/m eingestellt.

### Vergleichsbeispiel B

Unter Verwendung eines Blasfolien-Coextrusionswerkzeugs analog zu Beispiel 1 wurde eine zweischichtige Folie mit einer Nennschichtdickenabfolge 20 µm, 60 µm geformt. Die 60 µm dicke Schicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für diese Schicht verwendeten EVA-Copolymerisats, einem MFI von 1,8 g/10 min, gemessen nach DIN 53 735 bei 190 °C mit einer Prüfbelastung von 2,16 kg, und einer Dichte von 0,926 g/cm³ gebildet. Diese Schicht wurde auf dem Coextrusionsblaskopf als Aussenschicht gewählt. Das EVA-Polymerisat wurde mit einer Massetemperatur von 190 °C verarbeitet.

Die 20 µm dicke Innenschicht der Folie wurde aus einem Blend bestehend aus 80 Gew.-% des für die 60 µm dicke Schicht eingesetzten EVA-Copolymeren und 20 Gew.-% eines Styrolpolymerisats hergestellt. Das Styrolpolymerisat hatte eine Dichte von 1,05 g/cm3 und einem MFI von 4,0 g/10min, gemessen nach DIN 53 735 bei 200 °C und einer Prüfbelastung von 5 kg. Das Polymer-Blend wurde in der Innenschicht mit einer Massetemperatur von 205 °C verarbeitet.

Die so hergestellte Folie wurde an ihrer Aussenseite einer Corona-Behandlung unterzogen und so eine Oberflächenspannung der nicht mattierten Seite der Folie von 38 mN/m eingestellt.

### Vergleichsbeispiel C

Ein nach dem Stand der Technik bekannter zweischichtiger Aufbau wurde durch Beschichtung einer einschichtigen Folie mit einer polyolefinischen Beschichtungsmasse auf einer mattierten Beschichtungswalze mit einer Prägetiefe von 16 µm erhalten. Die einschichtige Folie wurde aus einem EVA-Copolymer mit einem VA-Anteil von 5 Gew.-%, einem MFI von 1,8 g/10 min, gemessen nach DIN 53 735 bei 190°C mit einer Prüfbelastung von 2,16 kg, und und einer Dichte von 0,926 g/cm³ erzeugt. Sie wurde mittels eines Blasfolienwerkzeugs in einer Dicke von 55 µm hergestellt. Die Matt-Beschichtung erfolgte mit einem Polyethylen mit niedriger Dichte von 0,915 glcm3 und einem MFI von 8 g/10 min, gemessen nach DIN 53735 bei 190°C mit einer Prüfbelastung von 2,16 kg. Die Beschichtung erfolgte mit einer Nenndicke von 25 µm.

Prüfungen an den im Rahmen der Beispiele und Vergleichsbeispiele hergestellten Mustern:

### Bestimmung von R₇ und R _{Max}

Die gemittelte Rauhtiefe R_{z} und die maximale Rauhtiefe R_{MAX} sind durch DIN 4768 definiert. Sie wurden nach dem Tastschnittverfahren entlang einer Taststrecke senkrecht zur Maschinenlaufrichtung der Folien an der mattierten Folienseite bestimmt. Die Vermessung erfolgte mit einem Perthometer-Messgerät der Fa. Feinprüf Perthen, Göttingen.

### Bestimmung der Glanzzahl

Die dimensionslose Glanzzahl wurde nach DIN 67 530 durch Reflektometermessung bestimmt.

### Bestimmung der Trübung

Die Trübung wurde ausschließlich an den nicht eingefärbten Proben nach ASTM D 1003 bestimmt.

### Bestimmung der Reißkraft und -dehnung sowie der Kraft für 10% Dehnung

[0071 ] Reißkraft und -dehnung sowie die Kraft für eine Dehnung von 10% wurden nach DIN 53 544 an 15 mm breiten Prüfstreifen ermittelt.

### Bestimmung der Rollneigung

Die Rollneigung wurde an Kreuzschnitten bestimmt. Hierzu wurden zwei 113 mm lange, sich unter einem Winkel von 90° schneidende Schnitte, die jeweils einen 45° Winkel mit der Maschinenlaufrichtung der Folie einschlossen und in ihren Abmessungen symmetrisch zum gegenseitigen Schnittpunkt sind, in die Proben eingebracht. Die Beurteilung erfolgte durch die Messung des größten Abstandes der sich einrollenden Spitzen des Kreuzschnitts.

### Wertung der Eigenschaften der erfindungsgemäßen Folien

Aus Tabelle 1 ist unschwer zu erkennen, daß die erfindungsgemäßen Folien aus den Beispielen 1 bis 5 den in den Vergleichsbeispielen A bis C beschriebenen Folien hinsichtlich des komplexen, dür die Anwendung als Trägerfilm für Wundpflaster zu erfüllenden Anforderungsprofils überlegen sind.

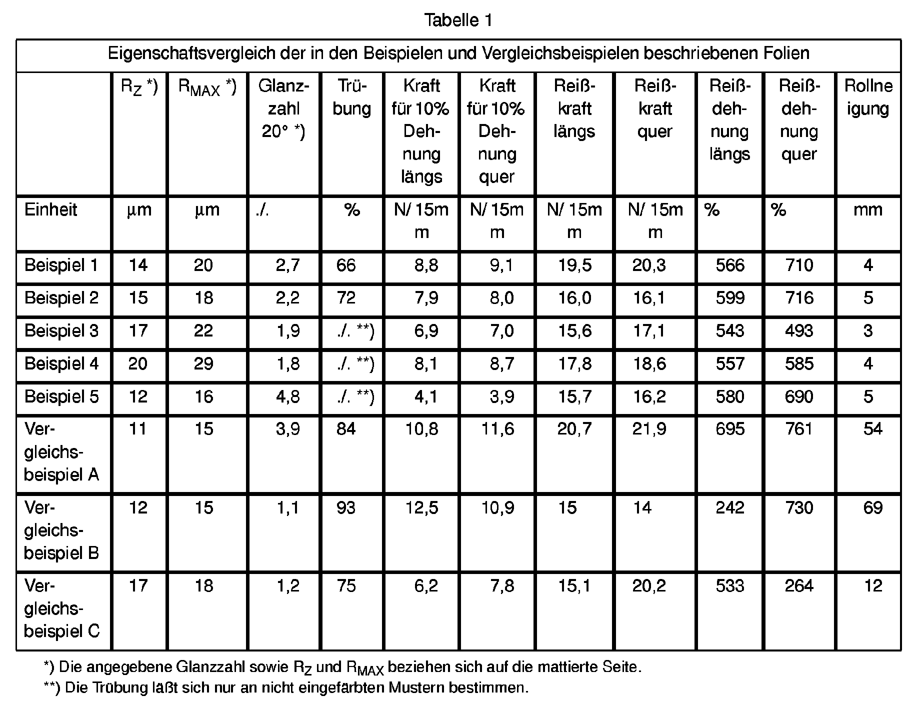

Sämtliche in den Beispielen aufgeführten erfindungsgemäß verwendeten Folien weisen im Vergleich zu den im Rahmen der Vergleichsbeispiele untersuchten Folien eine minimale Rollneigung auf, was auf eine minimale Beeinflussung des thermischen Ausdehnungsverhaltens der Schichtsilikat-gefüllten Schicht durch den Füllstoff hinweist.

Die Unterschiede in der Rollneigung werden sowohl im Vergleich mit mattbeschichteten als auch mit organischen Mattierungsmitteln gefüllten mehrschichtigen Folien deutlich. Gegenüber den matt beschichteten und mit Polystyrol mattierten Vergleichsmustern lassen sich zudem deutlich homogenere, d.h. in der Folienebene weitestgehend isotrope, mechanische Eigenschaften der Folie erzielen, was eine zusätzliche Anwendungs-Sicherheit schafft.

Die Folien lassen sich durch die breitere Auswahl an weicheren Rohstoffen, die für den Blasfolienprozeß in Frage kommen, zudem über einen weiten Bereich in ihren elastischen Eigenschaften verändern, was im Falle der herkömmlichen Beschichtung nicht möglich ist, da sich Beschichtungsanlagen i.a. nicht mit den für die Verarbeitung solcher Materialien notwendigen geringen Bahnspannungen betreiben lassen.

Schichtsilikat-Teilchen der beschriebenen Korngröße können die gewünschte Mattigkeit hervorrufen, wobei sich durch ihre definierte Korngröße die geforderten Glanzzahlen bzw. Oberflächen-Rauhigkeiten ausgezeichnet reproduzieren lassen. Die Mattierung durch Talkum bewirkt zudem eine nur geringe Erhöhung der Trübung, so daß sich auch transluzente Folien realisieren lassen.

## Patentansprüche

1. Verwendung einer mindestens zweischichtigen, asymmetrischen, matten Polyolefin-Folie, die eine geringe Rollneigung aufweist und einseitig Glanzwerte keiner 5, gemessen nach DIN 67 530 unter einem Winkel von 20°, besitzt, wobei die die geringeren Glanzwerte aufweisende Aussenschicht der Folie einen Anteil von 0 - 25 % an im wesentlichen gleichmäßig verteiltem Schichtsilikat-Füllstoff enthält, dessen mittlere Korngröße kleiner 20 I-Lm beträgt und als Matrixmaterialien der einzelnen Schichten der Polyolefinharz-Schichtstruktur Olefin-Polymerisate umfassend Olefin-Homopolymerisate, Olefin-Copolymerisate und Olefin-Copolymerisate mit Sauerstoff-Atome enthaltenden Comonomeren, wobei die Polyolefinharze Schmelzflußindices zwischen 0,2 g/10 min und 8,0 g/10 min, gemessen nach DIN 53 735 bei 190°C und einer Prüfbelastung von 2,16 kg, aufweisen und ihre Dichte zwischen 0,900 g/cm³ und 0,960 g/cm³ liegt, und die wesentlichen Eigenschaften der Matrixmaterialien der einzelnen Schichten gleich sind, als Trägerfilm für medizinische Pflaster.

2. Verwendung der Folie nach Anspruch 1, dadurch gekennzeichnet, daß das Schichtsilikat Talkum als mattierendes Mittel in eine der Außenseiten der erfindungsgemäßen Folien eingearbeitet wird.

3. Verwendung der Folie nach den Ansprüchen 1 und/oder 2, dadurch gekennzeichnet, daß die einzelnen Schichten der Folie weitere Verarbeitungshilfsmittel enthalten können, wobei Silikate, Wachse und Farben zu den bevorzugten Additiven zählen.

4. Verwendung der Folie nach den Ansprüchen 1 und/oder 2 und/oder 3, dadurch gekennzeichnet, daß die Zugabe von Schichtsilikat in Form eines Additiv-Batches erfolgt, wobei als Polymer-Harz-Basis für dieses Additiv-Batch bevorzugt Olefin-Polymerisate eingesetzt werden, deren Schmelzflußindices größer sind als die der jeweiligen für die einzelnen Schichten der erfindungsgemäßen Schichtstruktur verwendeten Matrixharze, in besonderem Maß geeignet sind Polyolefinharze mit Schmelzflußindices von 8,0 g/10 min bis 24,0 g/10 min, gemessen nach DIN 53 735 bei 190°C und einer Prüfbelastung von 2,16 kg.

5. Verwendung der Folie nach einem der Ansprüche 1 bis 4, dadurch gekennzeichnet, daß die Folie durch einen Blasfolienprozeß hergestellt wurde.

6. Verwendung der Folie nach einem der Ansprüche 1 bis 5, dadurch gekennzeichnet, daß die Folie eine Gesamtschichtdicke von mindestens 40 µm und höchstens 200 wm, besonders bevorzugt mindestens 50 µm und höchstens 100 µm besitzt.

7. Verwendung der Folie nach einem der Ansprüche 1 bis 6, dadurch gekennzeichnet, daß die mattierte Schicht einen Anteil von 15 bis 40 % an der Gesamtdicke der Folie aufweist.

8. Verwendung der Folie nach wenigstens einem der vorangehenden Ansprüche, dadurch gekennzeichnet, daß die Folie ein- oder beidseitig wenigstens einem der bekannten chemischen und/oder physikalischen Oberflächenbehandlungsverfahren unterzogen wurde.

## Claims

1. The use of an at least two-layer, asymmetrical, matt polyolefin film which has a low tendency to curl and has on one side gloss values of less than 5, measured in accordance with DIN 67 530 at an angle of 20°, wherein the outer layer of the film having the lower gloss values contains a proportion from 0 - 25% of substantially uniformly dispersed layer silicate filler, the average particle size of which is less than 20 m and, as matrix materials of the individual layers of the polyolefin resin layer structure, olefin polymers including olefin homopolymers, olefin copolymers and olefin copolymers with comonomers containing oxygen atoms, wherein the polyolefin resins have melt flow indices between 0.2 g/10 min and 8.0 g/10 min, measured in accordance with DIN 53 735 at 190°C and with a test load of 2.16 kg, and their density is between 0.900 glcm³ and 0.960 g/cm³, and the principal properties of the matrix materials of the individual layers are the same, as support film for medical plasters.

2. The use of the film according to claim 1, characterised in that the layer silicate talc is incorporated as flatting agent in one of the outer sides of the films according to the invention.

3. The use of the film according to claim 1 and/or 2, characterised in that the individual layers of the film may contain further processing aids, the preferred additives including silicates, waxes and colorants.

4. The use of the film according to claim 1 and/or 2 and/or 3, characterised in that the addition of layer silicate takes place in the form of an additive batch, the preferred polymer-resin basis used for said additive batch being olefin polymers, the melt flow indices of which are greater than those of the matrix resins used in each case for the individual layers of the layer structure according to the invention, particularly suitable polyolefin resins having melt flow indices from 8.0 g/10 min to 24.0 g/1 0 min, measured in accordance with DIN 53 735 at 190°C and with a test load of 2.16 kg.

5. The use of the film according to one of claims 1 to 4, characterised in that the film was manufactured by a blown film process.

6. The use of the film according to one of claims 1 to 5, characterised in that the film has a total layer thickness of at least 40 µm and at most 200 wm, particularly preferably at least 50 µm and at most 100 µm.

7. The use of the film according to one of claims 1 to 6, characterised in that the flatted layer has a proportion from 15 to 40% of the total thickness of the film.

8. The use of the film according to at least one of the preceding claims, characterised in that the film, on one or both sides, underwent at least one of the known chemical and/or physical surface treatment processes.

## Revendications

1. Utilisation d'une feuille mate de polyoléfine, asymétrique, comprenant au moins deux couches, qui présente une faible tendance au gondolage et qui possède, sur une face, une valeur de brillant inférieure à 5, mesurée conformément à la norme DIN 67 530 sous un angle de 20°, dans laquelle la couche externe de la feuille présentant la valeur de brillant inférieure possède une fraction à concurrence de 0 à 25% d'une matière de charge de silicate stratifié répartie de manière essentiellement uniforme, dont la granulométrie moyenne est inférieure à 20 wm, et contient, à titre de matières matricielles des couches individuelles de la structure stratifiée en résine de polyoléfine, des polymères d'oléfines englobant des homopolymères d'oléfines, des copolymères d'oléfines et des copolymères d'oléfines avec des comonomères contenant des atomes d'oxygène, les résines de polyoléfines présentant des indices de fusion entre 0,2 g/10 min et 8,0 g/10 min, mesurés conformément à la norme DIN 53 735 à 190°C et avec une charge d'essai de 2,16 kg, leur densité s'élevant entre 0,900 g/cm³ et 0,960 g/cm³, les propriétés essentielles des matières matricielles des couches individuelles étant égales, à titre de film de support pour des emplâtres médicaux.

2. Utilisation de la feuille selon la revendication 1, caractérisée en ce que le silicate stratifié représente du talc que l'on incorpore à titre d'agent de matage dans une des faces externes des feuilles selon l'invention.

3. Utilisation de la feuille selon les revendications 1 et/ou 2, caractérisée en ce que les couches individuelles de la feuille peuvent contenir des adjuvants de traitement supplémentaires, des silicates, des cires et des colorants faisant partie des additifs préférés.

4. Utilisation de la feuille selon les revendications 1 et/ou 2 et/ou 3, caractérisée en ce que l'addition du silicate stratifié a lieu sous la forme d'une charge d'additif, dans laquelle on met en oeuvre, à titre de base de résine polymère pour cette charge d'additif, de préférence des polymères d'oléfines dont les indices de fusion sont supérieurs à ceux des résines matricielles utilisées respectivement pour les couches individuelles de la structure de couches selon l'invention; sont appropriées dans une mesure particulière, des résines de polyoléfines possédant des indices de fusion de 8,0 g/10 min à 24,0 g/10 min mesurés conformément à la norme DIN 53 735 à 190°C et avec une charge d'essai de 2,16 kg.

5. Utilisation de la feuille selon l'une quelconque des revendications 1 à 4, caractérisée en ce qu'on fabrique la feuille via un procédé d'extrusion-soufflage.

6. Utilisation de la feuille selon l'une quelconque des revendications 1 à 5, caractérisée en ce que la feuille possède une épaisseur de couche totale minimale de 40 µm et maximale de 200 µm, de manière particulièrement préférée minimale de 50 µm et maximale de 100 wm.

7. Utilisation de la feuille selon l'une quelconque des revendications 1 à 6, caractérisée en ce que la couche matée présente une fraction représentant de 15 à 40% de l'épaisseur totale de la feuille.

B. Utilisation de la feuille selon au moins une des revendications précédentes, caractérisée en ce qu'on soumet la feuille, sur une seule face ou sur les deux faces, au moins à un des procédés de traitement superficiels chimiques et/ou physiques connus.
